# EUROPEAN PATENT APPLICATION

(11) **EP 2 277 851 A1**
(43) Date of publication of application: **26.01.2011**
(21) Application number: 09178833.1
(22) Date of filing: 11.12.2009
(51) Int. Cl.: C07C 231/02, C07C 237/46

(54) **Acetylation using reduced volume of acetic acid anhydride for synthesizing non-ionic X-ray contrast agents**

(30) Priority: 21.07.2009 US 227087 P
(71) Applicant: GE Healthcare AS, 0401 Oslo (NO)
(72) Inventor: Cervenka, Jan, N-0401, Oslo (NO); Holmaas, Lars Terje, N-4521, Spangereid (NO); Larsen, Leif, N-4521, Spangereid (NO)
(74) Representative: Wulff, Marianne Weiby

(57) **Abstract**

This invention relates to an improved method for the synthesis of 5-acetamido-N,N'-bis(2,3-dihydroxypropyl)-2,4,6-triiodoisophthalamide ("Compound A"), an intermediate in the industrial preparation of non-ionic X-ray contrast agents. In particular, it relates to acetylation of 5-amino-N, N'-bis(2,3-dihydroxypropyl)-2,4,6-triiodo-1,3-benzenedicarboxamide ("Compound B") using a reagent and solvent mixture containing between about 1.5 and about 3.0 liter of acetic anhydride and acetic acid per kilogram of Compound B. Preferably, following the acetylation reaction, the reagent and solvent mixture is distilled for re-use.

## Description

### TECHNICAL FIELD

This invention relates generally to large-scale synthesis of non-ionic X-ray contrast agents. It further relates to an improved method for the synthesis of 5-acetamido-N,N'-bis(2,3-dihydroxypropyl)-2,4,6-triiodoisophthalamide ("Compound A"), an intermediate in the industrial preparation of non-ionic X-ray contrast agents. In particular, it relates to acetylation of 5-amino-N, N'-bis(2,3-dihydroxypropyl)-2,4,6-triiodo-1,3-benzenedicarboxamide ("Compound B") using a reduced amount of acetic anhydride and acetic acid as a reagent and solvent mixture.

### BACKGROUND OF THE INVENTION

Non-ionic X-ray contrast agents constitute a very important class of pharmaceutical compounds produced in large quantities. 5-[N-(2,3-dihydroxypropyl)-acetamido]-N,N'-bis(2,3-dihydroxypropyl)-2,4,6-triiodo-isophthalamide ("iohexol"), 5-[N-(2-hydroxy-3-methoxypropyl)acetamido]-N,N'-bis(2,3-dihydroxypropyl)-2,4,6-triiodo-isophthalamide ("iopentol") and 1,3-bis(acetamido)-N,N'-bis[3,5-bis(2,3-dihydroxypropyl-aminocarbonyl)-2,4,6-triiodophenyl]-2-hydroxypropane ("iodixanol") are important examples of such compounds. They generally contain one or two triiodinated benzene rings.

In particular, iodixanol, marketed under the trade name Visipaque®, is one of the most used agents in diagnostic X-ray procedures. It is produced in large quantities by GE Healthcare in Lindesnes, Norway. The industrial production of iodixanol involves a multistep chemical synthesis as shown in Scheme 1 below. To reduce the cost of the final product, it is critical to optimize each synthetic step. Even a small improvement in reaction design can lead to significant savings in a large scale production.

The instant improvement is directed to the acetylation step, where 5-amino-N,N'-bis(2,3-dihydroxypropyl)-2,4,6-triiodoisophthalamide (Compound B) is acetylated to produce 5-acetylamino-N,N'-bis(2,3-dihydroxypropyl)-2,4,6-triiodoisophthalamide (Compound A) using acetic anhydride as the acetylating reagent. See Scheme 1 below. According to the present invention, the reagent volume as well as the solvent volume used in the acetylation reaction are significantly reduced.

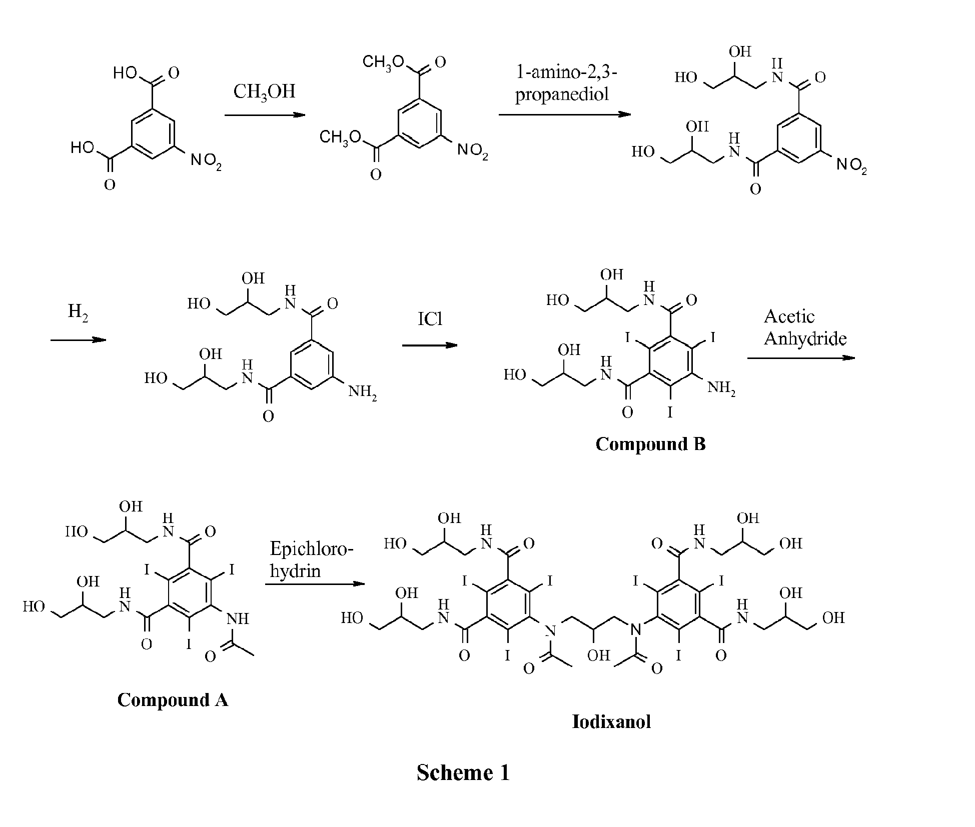

### SUMMARY OF THE INVENTION

The present invention provides an industrial process for preparing Compound A by acetylation of Compound B. Specifically, it uses between about 1.5 and about 3.0 liter of acetic anhydride and acetic acid as a reagent and solvent mixture per kilogram of Compound B. In a preferred embodiment, following the acetylation reaction, the reagent and solvent mixture are distilled for re-use in a subsequent acetylation reaction from Compound B to Compound A.

### DETAILED DESCRIPTION OF THE INVENTION

In the acetylation reaction of Compound B, both the amino group and the hydroxyl groups are acetylated. While the hydroxyl groups are later deacetylated by the addition of aqueous sodium hydroxide, a full acetylation of Compound B involves the addition of six acetic anhydride equivalents (four O-acetyls and two N-acetyls from four hydroxyl groups and two amino hydrogens respectively). See Scheme 2. Typically, acetic anhydride is also used as the solvent in the acetylation reaction to avoid introducing an additional component in the system. In such a design where no other solvent is present, the volume of acetic anhydride in the acetylation reaction typically exceeds more than 3.0 L per kilogram of Compound B.

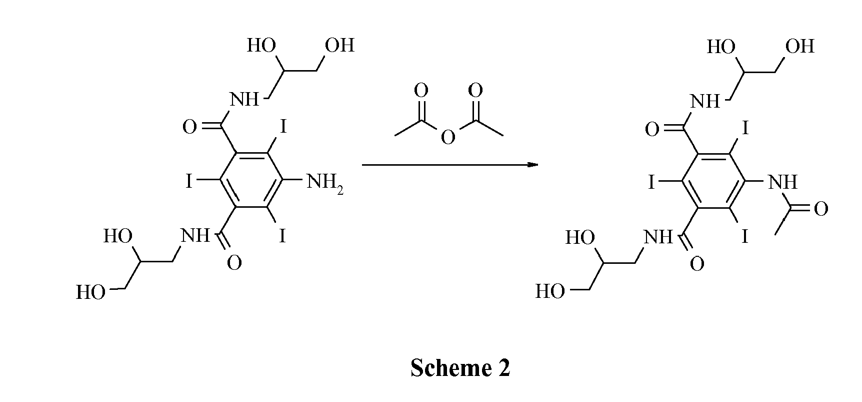

We have now surprisingly found that such high amount of acetic anhydride is not required to complete the acetylation reaction. The reaction can be led to completion using a blend of acetic anhydride and acetic acid with the latter as a solvent. In the instant method, the volume requirement for reagent and solvent in the acetylation reaction can be significantly reduced from the volume of acetic anhydride that has been previously used. Specifically, a combined volume of acetic anhydride and acetic acid between about 1.5 and about 3.0 liter per kilogram of Compound B can be safely and effectively operated to complete the acetylation reaction. In a preferred embodiment, the volume range is about 1.5 and about 2.0 liter of acetic anhydride and acetic acid per kilogram of Compound B.

Under the present procedure, the acetylation reaction solution is still stirrable. The exothermic nature in the acetylation reaction can be handled safely, and a full conversion from Compound B to Compound A may be obtained. The combination of acetic anhydride and acetic acid further allows the excess reagent and solvent mixture to be regenerated and re-used in another batch of acetylation reaction.

The temperature of the reaction is typically in the range of about 50 to about 125°C, gradually increasing as the exotherm in the reaction heats the reaction mixture. In certain embodiments, the acetylation reaction is preferably started at ambient temperature. An acid catalyst, such as sulphuric acid or p-toluene sulfonic acid, may also be added in the acetylation reaction.

The instant invention provides several distinct advantages. First, the amount of expensive acetic anhydride consumed per batch is reduced. In addition, because acetic acid is cheaper than acetic anhydride, the partial replacement of acetic anhydride with acetic acid lowers the reagent cost and in turn drives down the overall production cost for the drug product.

Second, the cost of manufacture is further lowered because energy costs and process time are reduced due to less volume of acetic anhydride and acetic acid to be processed after the acetylation reaction.

Finally, the instant method allows for a substantial amount of the excess acetic anhydride and acetic acid to be regenerated and re-used after the completion of the acetylation reaction. In a traditional approach where only acetic anhydride is used without acetic acid, reagent regeneration is impractical. Specifically, if pure acetic anhydride is used in the reaction, it is necessary to carry out each batch of acetylation with 100% acetic anhydride. Thus, any regeneration of excess acetic anhydride requires a complete separation of acetic anhydride from acetic acid, the inevitable byproduct of the acetylation reaction (one mole of acetic anhydride gives one mole of acetylated product and one more of acetic acid). This separation involves a series of difficult and expensive processes.

The instant process, on the other hand, begins with the use of a mixture of acetic anhydride and acetic acid as a reagent and solvent mixture in the first batch and continues to use a mixture of acetic anhydride and acetic acid in subsequent batches. Thus, excess acetic anhydride and acetic acid are mixed with acetic acid byproduct from the acetylation reaction and the remixed acetic anhydride and acetic acid can be selected for regeneration and re-use for new batches of acetylation reaction. For example, after the acetylation reaction, excess acetic anhydride and acetic acid may be regenerated by distillation because acetic acid has a lower boiling point than acetic anhydride (118°C vs. 136°C at atmospheric pressure). As shown in the examples below, a fraction of the distillate may be re-used in the next batch of acetylation reaction and mixed with fresh acetic anhydride to form the desired total amount of reagent and solvent mixture.

The invention is illustrated further by the following examples that are not to be construed as limiting the invention in scope to the specific procedures or products described in them.

### EXAMPLES

### EXAMPLE 1

Compound B (5356 kg) was suspended in a mixture of acetic anhydride and acetic acid (10900 L, 2.0 L/kg Compound B, 91 v/v % acetic anhydride and 9 v/v % acetic acid) at ambient temperature. This mixture was prepared by mixing acetic anhydride (8115 kg) and regenerated acetic anhydride (3460 kg), the latter containing 66 v/v % acetic anhydride and 34 v/v % acetic acid obtained from a previous batch. The mixture was heated to 55°C. Then, p-toluene sulfonic acid (25 kg) was added and the reaction mixture was further heated to 117°C over about 90 minutes. All solids were dissolved in the reaction, and 100 % of Compound B was acetylated. The remaining acetic anhydride/acetic acid (8430 L) was distilled off under reduced pressure resulting in a highly viscous reaction mixture. Forty-one percent of the distillate could be re-used as reagent in a later batch without a discrete purification step.

### EXAMPLE 2

Compound B (5356 kg) was suspended in a mixture of acetic anhydride and acetic acid (7760 L, 1.45 L/kg Compound B, 87 v/v % acetic anhydride and 13 v/v % acetic acid) at ambient temperature. This mixture was prepared by mixing acetic anhydride (6086 kg) and regenerated acetic anhydride (2295 kg), the latter containing 48 v/v % acetic anhydride and 52 v/v % acetic acid obtained from a previous batch. The mixture was heated to 55°C. Then, p-toluene sulfonic acid (25 kg) was added and the reaction mixture was further heated to 124°C over about 90 minutes. All solids were dissolved in the reaction, and 100 % of Compound B was acetylated. The remaining acetic anhydride/acetic acid (5580 L) was distilled off under reduced pressure resulting in a very viscous reaction mixture. Thirty-nine percent of the distillate could be re-used as reagent in a later batch without a discrete purification step.

All patents, journal articles, publications and other documents discussed and/or cited above are hereby incorporated by reference.

## Claims

1. A process for industrial preparation of 5-acetamido-N,N'-bis(2,3-dihydroxypropyl)-2,4,6-triiodoisophthalamide ("Compound A") using between about 1.5 and about 3.0 liter of acetic anhydride and acetic acid as a reagent and solvent mixture per kilogram of 5-amino-N, N'-bis(2,3-dihydroxypropyl)-2,4,6-triiodo-1,3-benzenedicarboxamide ("Compound B") for acetylation of Compound B.

2. The process according to claim 1, wherein said reagent and solvent mixture is distilled, following the acetylation reaction, for re-use.

3. A process for industrial preparation of 5-acetamido-N,N'-bis(2,3-dihydroxypropyl)-2,4,6-triiodoisophthalamide ("Compound A") by acetylating 5-amino-N, N'-bis(2,3-dihydroxypropyl)-2,4,6-triiodo-1,3-benzenedicarboxamide ("Compound B") to completion using a blend of acetic anhydride and acetic acid with the latter as a solvent.

4. The process according to claim 3, wherein said blend of acetic anhydride and acetic acid is distilled, following the acetylation reaction, for re-use.
